# EUROPEAN PATENT SPECIFICATION

(11) **EP 1 529 550 B1**
(45) Date of publication and mention of the grant of the patent: **03.10.2007**
(21) Application number: 04026453.3
(22) Date of filing: 08.11.2004
(51) Int. Cl.: A61N 1/36, A61N 1/362, A61N 1/18

(54) **Electrotherapy apparatus**
Elektrotherapievorrichtung
Appareil électrothérapeutique

(30) Priority: 07.11.2003 EP 03025665
(43) Date of publication of application: 11.05.2005
(73) Proprietor: CardioLa Ltd., 8400 Winterthur (CH)
(72) Inventor: Lapanashvili, Larry, 8404 Winterthur (CH); Zimmermann, Thomas, 8203 Schaffhausen (CH)
(74) Representative: Manitz, Finsterwald & Partner GbR

(56) References cited:
- EP-A2- 0 573 946
- EP-A2- 0 774 273
- WO-A1-02/066112
- US-A- 5 063 929
- US-A- 5 111 816
- US-B1- 6 516 227

## Description

The present invention relates to an electrotherapy apparatus comprising at least one active electrode and at least one passive electrode and a circuit for generating biphasic pulses for application to said at least one active electrode, each said biphasic pulse having a generally rectangular positive half wave followed by a generally negative half wave, or vice versa, wherein the circuit has an output connected to said at least one active electrode and an output connected to said at least one passive electrode.

An electrotherapy apparatus of this kind is described in detail in the international patent application with the publication number WO 01/13990 A1. That publication describes the pulses that are preferably used as biphasic pulses and shows them having a rectangular positive half wave directly followed by a rectangular negative half wave and with an interval then being present until the biphasic pulse is repeated.

This is the preferred form of pulse used by the present applicants for electrotherapy, in particular counterpulsation electrotherapy. Unfortunately, it has been found that the circuits that are used to generate the biphasic pulses leads to the closing flank of the second half wave not being a vertical flank, but rather gradually decaying over a considerable period of time.

The object of the present invention is to provide a modified circuit which avoids this phenomenon and which leads to the closing flank of the second half pulse being essentially vertical.

In order to satisfy this object there is provided an electrotherapy apparatus of the initially named kind which is characterised in that a first switch is provided between said output to said at least one active electrode and said output to said at least one passive electrode and in that said circuit is adapted to close said first switch briefly at the end of the desired duration of the last half wave, typically the negative half wave of each biphasic pulse, said electrotherapy apparatus further comprising a transformer having a first centre connection at the primary side connected to a variable d.c. voltage source, there being second and third connections to said primary side of said transformer on either side of said first centre connection, said second connection being connectable via a second switch to a ground connection and said third connection being connectable via a third switch to a ground connection, wherein said output to said at least one active electrode comprises a connection to one end of a secondary winding of the transformer and said output to said passive electrode comprises a connection to a second end of said secondary winding of said transformer, wherein said microprocessor or said controller and/or said logic circuit is adapted to close first said second switch and then to open it again and to close the third switch and then open it again, said first switch being switched substantially coincident with the opening of said third switch.

By providing the first switch between the output to the at least one active electrode and the output to the at least one passive electrode and by closing this switch briefly at the end of the desired duration of the last half wave, which is typically the negative half wave of each biphasic pulse, the electrodes are briefly short-circuited and the closing flank of the second half pulse extends vertically as desired. It appears that the reason why the closing flank was previously not as steep as desired is to be found in a capacitive discharge phenomenon taking place within the body of the person or mammal being treated.

For the sake of completeness reference should also be made to US-A-5,063,929, to US-A-5,111,816, to US-A-6,516,227, to EP-A-0 774 273 and to EP-A-0 573 946. In US-A-5,063,929 an electrotherapy apparatus is described which also produces biphasic stimulation pulses, however, with an interval being present between the positive half wave and the negative half wave. In contrast to the present invention a transformer is not used in this reference to step up the input voltage or to generate the negative half wave. Accordingly, this reference does not deal with problems associated with a transformer, nor does it provide any suggestion how such problems could be solved. In the apparatus proposed in US-A-5,063,929 the negative half wave is produced by an H-bridge involving four transistors and is thus very different in principle from the present invention.

US-A-5,111,816 relates to a defibrillator/pacemaker and also uses an H-bridge. A transformer is provided, but is positioned before the H-bridge. This arrangement operates in accordance with a fundamentally different principle and has nothing to do with the circuitry provided by the present teaching.

US-A-6,516,227 describes a spinal cord stimulation system providing multiple stimulation channels, but does not involve a transformer for coupling the electrical stimulation to the person being treated. Instead, a series of capacitors is used. Again, the principle on which the apparatus is based is completely different from the presently claimed apparatus.

EP-A-0 774 273 relates to an apparatus and method for diagnosing, increasing the performance of and for restoring damaged nerve and muscle activities. The apparatus proposed there also does not involve a transformer.

Finally, EP-A-0 573 946 describes an electrical stimulator, i.e. an electrotherapy apparatus, and in one embodiment a transformer is used for the coupling to the patient and for the stepping up of the output voltage. However, the transformer is only operated in a unipolar manner. Moreover, there is no suggestion in the reference of the problem addressed by the present teaching, far less any suggestion as to how this problem can be solved.

Preferred embodiments of the present invention can be seen in the further claims and will be described in more detail with reference to the accompanying drawings in which:
- Fig. 1: shows a typical desired pulse form corresponding to Fig. 2B of the aforementioned document WO 01/13990 A1,
- Fig. 2: shows the actual pulse form which results when an electrical signal supposedly having the pulse form of Fig. 1 is applied to the human body,
- Fig. 3: shows a circuit for an electrotherapy apparatus in accordance with the present invention which corrects the pulse form of Fig. 2 so that it corresponds more closely to the desired pulse form in Fig. 1,
- Fig. 4: shows the actual pulse form as measured following use of the circuit in accordance with Fig. 3,
- Fig. 5: shows a typical electrocardiogram, and
- Fig. 6: shows a typical electrocardiogram with a train of biphasic pulses being triggered at the end of each T-wave.

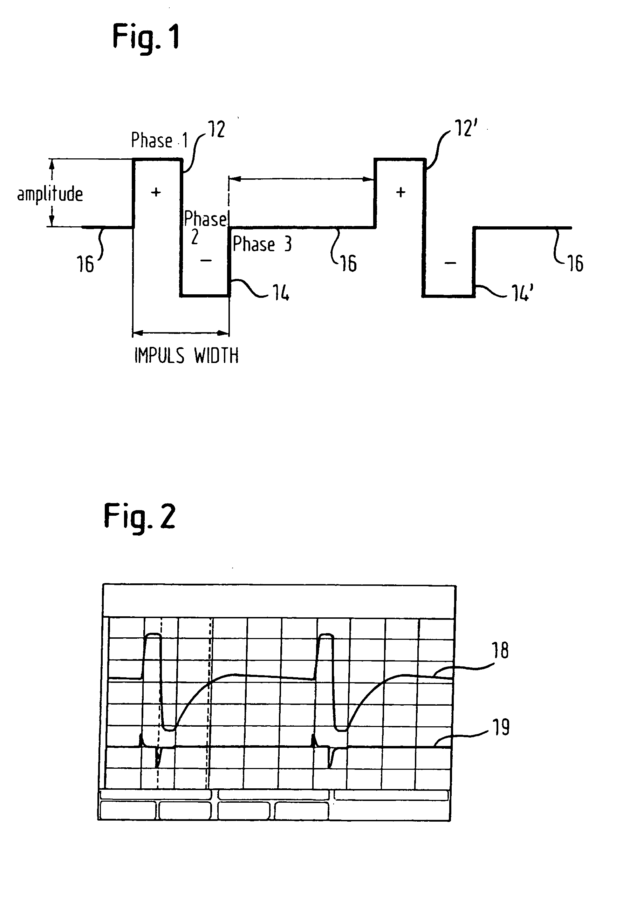

Turning first to Fig. 1 there can be seen the desired biphasic pulse form for use in electrotherapy. As shown in Fig. 1 the biphasic pulse form comprises a first positive half wave 12 followed by a second negative half wave 14 whereupon, at the end of the second negative half wave 14, the signal has zero amplitude until the next positive half wave 12', which is in turn followed immediately by a negative half wave 14', whereupon the signal form returns to the base line 16 with zero amplitude.

It can be seen from diagram of Fig. 1 that the amplitude of the positive half waves is the same as the amplitude of the negative half waves and that the pulse width comprises the total duration of the positive half wave 12 plus the negative half wave 14. The interval between the negative half wave 14 and the next positive half wave 12' is typically longer than the pulse width. In a practical embodiment the positive amplitude can readily amount to up to +50 volts, the negative amplitude can readily amount to - 50 V, the pulse width is conveniently selected to be 1 ms and the interval can vary in length from 1 ms to 25 ms or longer. In one preferred embodiment the stimulation applied by the apparatus comprises initially five biphasic pulses 12, 14 of 1 ms width with an interval of 5.666 ms between the negative half wave 14 of one pulse to the positive half wave 12' of the next pulse and this initial train of five pulses is supplemented by a further train of pulses having the same pulse width, but with a longer interval of say 24 ms between the individual pulses. The pulse train is conveniently triggered to start at a time corresponding to the predicted end of the T-wave, or at least at a time within a window extending from -5 % of the R-R path length of the last completed heart cycle prior to the predicted time of the T-wave (predicted using the Bazett relationship) up to +45 % of the R-R path of the previous heart cycle after the predicted end of the T-wave. The train of biphasic pulses is stopped preferably at a time such that the muscle contraction produced by the stimulation has terminated before the next R peak is detected. Instead of using the R-R value from the preceding heart cycle, it is also possible to base the timing on an average R-R value determined over the last few heart cycles, with any heart cycles that are unduly short or unduly long being omitted from the averaging process, or on another representative value.

Precise timing schemes for such biphasic pulses are, for example, given in the simultaneously filed patent application with the title: Electrotherapy Apparatus And Method Of Treating A Person Or A Mammal Using Such Electrotherapy Apparatus (Attorney's file: C5148PWO) the content of which is incorporated into the present application by reference and in the further simultaneously filed patent application with the same title (Attorney's file C5147PWO), the content of which is also incorporated into the present application by reference.

Fig. 2 reproduces at the top an oscilloscope trace 18 which shows how the potential changes at an active electrode applied to the surface of a body of a human being overlying a muscle which is to be stimulated with the biphasic pulse. It can be seen that the positive half wave of the pulse applied to the electrode closely follows the desired signal shape of Fig. 1 but that the negative half wave has a closing flank which decays only gradually and takes a considerable time to reach the baseline value. Moreover, the signal trace is elevated above the baseline for a period.

The bottom curve 19 shows the currents which flow during generation of the biphasic pulses.

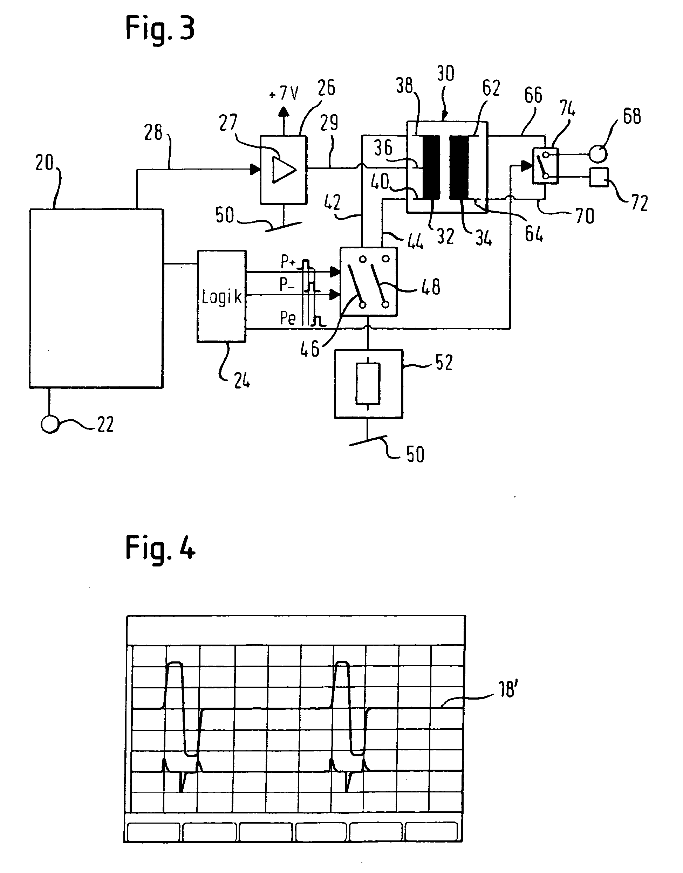

Turning now to Fig. 3 there can be seen a circuit diagram explaining how the invention achieves the desired rectangular biphasic pulse as shown in Fig. 1 (with the actual results being shown in Fig. 4) and which gives insight into how these rectangular pulses are generated.

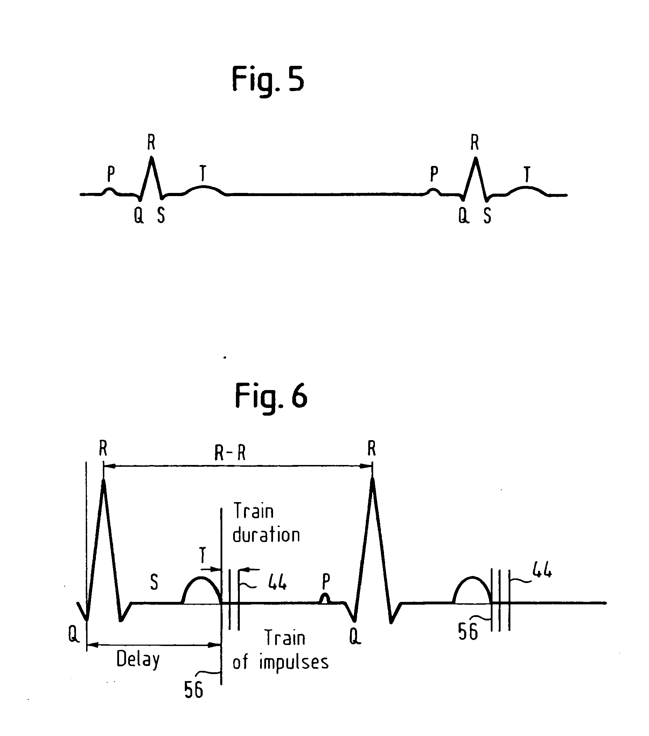

Reference numeral 20 refers to a microprocessor or controller which receives signals from a sensor 22 representing a patient's heart beat. The sensor 22 can, for example, be an electrocardiograph which produces the typical signal shown in Fig. 5. With such a signal the R peaks are generally considered as input signals for the operation of the microprocessor 20 because they are the largest signals and the easiest to recognise. The R-R path length is directly proportional to the patient's heart rate so that, with a pulse of 60 beats per minute, the distance between a pair of succeeding R-R peaks is 1000 ms.

Shown in Fig. 5 at T is the so-called T-wave, the end of which marks the boundary between the systolic phase of the heart cycle and the diastolic phase. The microprocessor 20 calculates from the last two detected R peaks the distance between them in ms and predicts on the basis of this measurement when the end of the T-wave in the next heart cycle will occur. This prediction is done using the so-called Bazett relationship which enables the end of the T-wave to be predicted from the last measured R-R path length.

Once the end of the T-wave has been predicted, the microprocessor sends to a logic circuit 24 signals which permit the forming of single rectangular biphasic pulses 12, 14 as shown in Fig. 1. The timing of the biphasic pulses is controlled by the microprocessor, in other words the microprocessor triggers the biphasic pulses.

The circuit of Fig. 3 basically operates as follows:

The item 26 represents a d.c. power source including an amplifier 27 which provides a d.c. output voltage on line 29, in this example in the range from 0 to 5 V. The level of the output voltage is controlled by the microprocessor via the line indicated by 28. The reference numeral 30 designates a transformer having a primary winding 32 and a secondary winding 34. The output of the d.c. source 26 is fed to a centre terminal 36 on the primary winding 32 of the transformer. The primary winding of the transformer has two further terminals 38 and 40 which are connected via respective lines 42 and 44 and respective switches 46 and 48 to a ground connection 50. Box 52 represents a safety circuit which checks that both switches 46 and 48 are not closed at the same time.

The secondary winding 34 of the transformer 30 has two terminals 62 and 64 in this embodiment, the output terminal 62 being connected via suitable leads 66 to an active electrode 68 overlying a muscle to be stimulated on a person's body whereas the output terminal 64 is connected via a suitable lead 70 to a passive electrode 72 which is placed on the person's body in proximity to the active electrode so that the patient senses the stimulating pulses applied to the active electrode. The reference numeral 74 designates a switch provided between the circuit output 62 to the active electrode 68 and the circuit output 64 to the passive electrode 72.

Once the microprocessor 20 commands the triggering of a biphasic pulse, the logic circuit 22 sends a first rectangular pulse P+ to the said switch 46 which connects the output terminal 38 of the transformer to ground inducing an amplified positive half wave at the output terminal 62 of the secondary winding 34 of the transformer which is applied to the person being treated via the active electrode 68. As soon as the trailing flank of the P+ pulse from the logic circuit in Fig. 3 arrives, the switch 46 opens again. That is to say the width of the P+ pulse determines the duration of the positive half wave of the biphasic pulse. Immediately thereafter the logic circuit sends a second pulse indicated as P- in Fig. 3 to the third switch 48 which closes and connects the terminal 40 of the primary winding 32 of the transformer to ground. This induces the subsequent negative half wave at the secondary winding of the transformer, which is again applied via the terminal 62 of the secondary winding, the lead 66 and the active electrode 68 to the patient. The trailing flank of the P- pulse opens the switch 48 again so that the peak width of the negative half wave is determined by the P- pulse.

The invention now provides a further pulse Pe which is triggered immediately following the P- pulse and which serves to close the first switch 74 provided between the output to the active electrode and the output to the passive electrode. This switch is shut for a short time corresponding to the width of the pulse Pe and actively discharges the active electrode to the passive electrode and thus to ground, since the passive electrode is grounded. The result can be seen in Fig. 4, the biphasic pulse applied to the patient shown by the top curve 18' corresponds closely to the desired biphasic pulse of Fig. 1. The width of the negative half wave is now substantially constant over its full height. The lower curve in Fig. 4 shows the current which flows when the switches 46, 48 and 74 are closed.

The transformer can increase the maximum input voltage from the d.c. source 26 of typically 5 V to an output voltage of typically 50 V by a suitable choice of the ratio of the number of windings at the primary side relative to the number of windings at the secondary side. If the input voltage is less than five volts, then the output voltage at the secondary side of the transformer is correspondingly smaller. The microprocessor is thus programmed to change the amplitude of the signals applied to the active electrode and to the passive electrode, for example by acting on the d.c. voltage source 26 via the line 28 to change the amplitude of the d.c. voltage applied to the centre terminal 36 at the primary side of the transformer. Moreover, the microprocessor is adapted to change the frequency of the pulses delivered at the output of the transformer. It does this by controlling the timing of the logic circuit which transmits the P+, P- und Pe pulses, which determines the pulse repetition frequency and the pulse interval of the electrical stimulation signals appearing at the output of the transformer which are applied to the active electrode.

It should be noted that all the switches 46, 48 and 74 in Fig. 3 are typically electronic switches. It should also be appreciated that the logic circuit 24 shown here as a discrete circuit could also be embodied in the microprocessor so that the microprocessor itself sends the P+, P- and Pe pulses with the correct timing to the electronic switches 46, 48 and 74 respectively.

If a plurality of active electrodes is provided then the desired signals can be fed to each electrode via a bank of switches provided at the output of the secondary side 34 of the transformer.

## Claims

1. Electrotherapy apparatus comprising at least one active electrode (68) and at least one passive electrode (72) and a circuit (20, 24, 26, 30, 46, 48) for generating biphasic pulses for application to said at least one active electrode, each said biphasic pulse having a generally rectangular positive half wave (12, 12') followed by a generally negative half wave (14, 14'), or vice versa, said circuit having an output (62) connected to said at least one active electrode (68) and an output (64) connected to said at least one passive electrode (72),
**characterised in that**
a first switch (74) is provided between said output (62) to said at least one active electrode (68) and said output (64) to said at least one passive electrode (72), **in that** said circuit is adapted to close said first switch briefly at the end of the desired duration of the last half wave (14, 14'), typically the negative half wave (14, 14') of each biphasic pulse, said electrotherapy apparatus further comprising a transformer (30) having a first centre connection (36) at the primary side (32) connected to a variable d.c. voltage source (26), there being second and third connections (38, 40) to said primary side (32) of said transformer (30) on either side of said first centre connection (36), said second connection (38) being connectable via a second switch (46) to a ground connection (50) and said third connection (40) being connectable via a third switch (48) to a ground connection (50), wherein said output (62) to said at least one active electrode (68) comprises a connection to one end of a secondary winding (34) of the transformer (30) and said output (64) to said passive electrode (72) comprises a connection to a second end of said secondary winding (34) of said transformer (30), wherein said microprocessor (20) or said controller and/or said logic circuit (24) is adapted to close first said second switch (46) and then to open it again and to close the third switch (48) and then open it again, said first switch (74) being switched substantially coincident with the opening of said third switch (48).

2. Electrotherapy apparatus in accordance with claim 1,
**characterised in that**
said first switch (74) is an electronic switch.

3. Electrotherapy apparatus in accordance with claim 1 or claim 2,
**characterised in that**
said circuit (20, 24, 26, 30, 46, 48) is a circuit receiving trigger inputs from a microprocessor (20), a microcontroller and/or a logic circuit (24) controlled by said microprocessor (20) and/or said controller.

4. Electrotherapy apparatus in accordance with claim 1,
**characterised in that**
said second and third switches (46, 48) are electronic switches.

5. Electrotherapy apparatus in accordance with one any of the preceding claims,
**characterised in that**
said d.c. voltage source (26) provides a d.c. output voltage of a variable level said level being controllable by said microprocessor (20) or said controller.

6. Electrotherapy apparatus in accordance with one any of the preceding claims,
**characterised in that**
the pulse repetition frequency and the pulse interval of the biphasic pulses is determined by the microprocessor (20) by determining the timing of the pulse signals P+, P- and Pe delivered by the logic circuit, with the width of the biphasic pulses being determined by the width of the signals P+, P- and Pe delivered by the logic circuit (24), which can be a result of the design of the logic circuit or a result of timing of the trigger signals issued by the microcontroller (20) to the logic circuit (24.

## Patentansprüche

1. Elektrotherapievorrichtung, umfassend mindestens eine aktive Elektrode (68) und mindestens eine passive Elektrode (72) und einen Schaltkreis (20, 24, 26, 30, 46, 48) zum Erzeugen von zweiphasigen Impulsen für eine Aufbringung auf die mindestens eine aktive Elektrode, wobei jeder zweiphasige Impuls eine im wesentlichen rechteckige positive Halbwelle (12, 12') gefolgt von einer im Wesentlichen negativen Halbwelle (14, 14') oder umgekehrt aufweist, wobei der Schaltkreis einen mit der mindestens einen aktiven Elektrode (68) verbundenen Ausgang (62) und einen mit der mindestens einen passiven Elektrode (72) verbundenen Ausgang (64) aufweist,
**dadurch gekennzeichnet, dass**
ein erster Schalter (74) zwischen dem Ausgang (62) zu der mindestens einen aktiven Elektrode (68) und dem Ausgang (64) zu der mindestens einen passiven Elektrode (72) vorgesehen ist, dass der Schaltkreis geeignet ist, um den ersten Schalter am Ende der gewünschten Dauer der letzten Halbwelle (14, 14'), typischerweise der negativen Halbwelle (14, 14') jedes zweiphasigen Impulses, kurz zu schließen, wobei die Elektrotherapievorrichtung ferner einen Transformator (30) umfasst, der eine erste Mittelverbindung (36) an der primären Seite (32) aufweist, die mit einer variablen DC-Spannungsquelle (26) verbunden ist, wobei zweite und dritte Verbindungen (38, 40) zu der primären Seite (32) des Transformators (30) an jeder Seite der ersten Mittelverbindung (36) vorhanden sind, wobei die zweite Verbindung (38) über einen zweiten Schalter (46) mit einem Masseanschluss (50) verbindbar ist und die dritte Verbindung (40) über einen dritten Schalter (48) mit einem Masseanschluss (50) verbindbar ist, wobei der Ausgang (62) zu der mindestens einen aktiven Elektrode (68) eine Verbindung zu einem Ende einer sekundären Wicklung (34) des Transformators (30) umfasst und der Ausgang (64) zu der passiven Elektrode (72) eine Verbindung zu einem zweiten Ende der sekundären Wicklung (34) des Transformators (30) umfasst, wobei der Mikroprozessor (20) oder der Controller und/oder der Logikschaltkreis (24) geeignet sind/ist, um den zweiten Schalter (46) zuerst zu schließen und ihn dann wieder zu öffnen und den dritten Schalter (48) zu schließen und ihn dann wieder zu öffnen, wobei der erste Schalter (74) im Wesentlichen gleichzeitig mit dem Öffnen des dritten Schalters (48) geschaltet wird.

2. Elektrotherapievorrichtung nach Anspruch 1,
**dadurch gekennzeichnet, dass**
der erste Schalter (74) ein elektronischer Schalter ist.

3. Elektrotherapievorrichtung nach Anspruch 1 oder 2,
**dadurch gekennzeichnet, dass**
der Schaltkreis (20, 24, 26, 30, 46, 48) ein Schaltkreis ist, der Auslöseeingänge von einem Mikroprozessor (20), einem Mikrocontroller und/oder einem Logikschaltkreis (24) empfängt, der durch den Mikroprozessor (20) und/oder dem Controller gesteuert ist.

4. Elektrotherapievorrichtung nach Anspruch 1,
**dadurch gekennzeichnet, dass**
der zweite und dritte Schalter (46, 48) elektronische Schalter sind.

5. Elektrotherapievorrichtung nach einem der vorangehenden Ansprüche,
**dadurch gekennzeichnet, dass**
die DC-Spannungsquelle (26) eine DC-Ausgangsspannung eines variablen Pegels liefert, wobei der Pegel durch den Mikroprozessor (20) oder den Controller steuerbar ist.

6. Elektrotherapievorrichtung nach einem der vorangehenden Ansprüche,
**dadurch gekennzeichnet, dass**
die Impulswiederholungsfrequenz und das Impulsintervall der zweiphasigen Impulse durch den Mikroprozessor (20) bestimmt werden, indem das Timing der durch den Logikschaltkreis gelieferten Impulssignale P+, P- und Pe bestimmt wird, wobei die Breite der zweiphasigen Impulse durch die Breite der durch den Logikschaltkreis (24) gelieferten Signale P+, P- und Pe bestimmt wird, was ein Ergebnis des Entwurfs des Logikschaltkreises oder ein Ergebnis des Timings der von dem Mikrocontroller (20) an den Logikschaltkreis (24) ausgegebenen Auslösesignale sein kann.

## Revendications

1. Appareil d'électrothérapie comprenant au moins une électrode active (68) et au moins une électrode passive (72) et un circuit (20, 24, 26, 30, 46, 48) pour générer des impulsions biphasées pour l'application à ladite au moins une électrode active, chacune desdites impulsions biphasées ayant une demi-onde positive généralement rectangulaire (12, 12') suivie par une demi-onde (14, 14') généralement négative, ou vice versa, ledit circuit ayant une sortie (62) connectée à ladite au moins une électrode active (68) et une sortie (64) connectée à ladite au moins une électrode passive (72),
**caractérisé en ce que**
un premier commutateur (74) est prévu entre ladite sortie (62) vers ladite au moins une électrode active (68) et ladite sortie (64) vers ladite au moins une électrode passive (72), **en ce que** ledit circuit est adapté à fermer ledit premier commutateur brièvement à la fin de la durée désirée de la dernière demi-onde (14, 14'), typiquement la demi-onde négative (14, 14') de chaque impulsion biphasée, ledit appareil d'électrothérapie comprenant en outre un transformateur (32) connecté à une source de tension continue variable (26), il est prévu une seconde et une troisième connexion (38, 40) vers le côté primaire (32) dudit transformateur (30) de part et d'autre de ladite première connexion centrale (36), ladite seconde connexion (32) pouvant être connectée via un second commutateur (46) à une connexion de masse (50), et ladite troisième connexion (40) pouvant être connectée via un troisième commutateur (43) à une connexion de masse (50), ladite sortie (62) vers ladite au moins une électrode active (68) comprend une connexion à une extrémité d'un enroulement secondaire (34) du transformateur (30) et ladite sortie (64) vers ladite électrode passive (72) comprend une connexion à une seconde extrémité dudit enroulement secondaire (34) dudit transformateur (30), ledit microprocesseur (20) ou ledit contrôleur et/ou ledit circuit logique (24) est adapté à fermer en premier ledit second commutateur (46) puis à l'ouvrir à nouveau, et à fermer le troisième commutateur (48) puis à l'ouvrir à nouveau, ledit premier commutateur (74) étant commuté sensiblement en coïncidence avec l'ouverture dudit troisième commutateur (48).

2. Appareil d'électrothérapie selon la revendication 1,
**caractérisé en ce que** ledit premier commutateur (74) est un commutateur électronique.

3. Appareil d'électrothérapie selon la revendication 1 ou 2,
**caractérisé en ce que** ledit circuit (20, 24, 26, 30, 46, 48) est un circuit recevant des entrées de déclenchement depuis un microprocesseur (20), un microcontrôleur et/ou un circuit logique (24) contrôlé par ledit microprocesseur (20) et/ou ledit contrôleur.

4. Appareil d'électrothérapie selon la revendication 1,
**caractérisé en ce que** ledit second et ledit troisième commutateur (46, 48) sont des commutateurs électroniques.

5. Appareil d'électrothérapie selon l'une quelconque des revendications précédentes,
**caractérisé en ce que** ladite source de tension continue (26) fournit une tension de sortie continue à un niveau variable, ledit niveau pouvant être contrôlé par ledit microprocesseur (20) ou ledit contrôleur.

6. Appareil d'électrothérapie selon l'une quelconque des revendications précédentes,
**caractérisé en ce que** la fréquence de répétition d'impulsions et l'intervalle d'impulsion des impulsions biphasées sont déterminés par le microprocesseur (20) en déterminant la temporisation des signaux par impulsion P +, P - et Pe fournis par le circuit logique, et la largeur des impulsions biphasées est déterminée par la largeur des signaux P +, P - et Pe fournis par le circuit logique (24), qui peuvent être un résultat de la conception du circuit logique ou un résultat de la temporisation des signaux de déclenchement délivrés par le microcontrôleur (20) vers le circuit logique (24).
